# EUROPEAN PATENT SPECIFICATION

(11) **EP 4 144 404 B1**
(45) Date of publication and mention of the grant of the patent: **10.04.2024**
(21) Application number: 21195050.6
(22) Date of filing: 06.09.2021
(51) Int. Cl.: A61N 1/05, A61N 1/375

(54) **ELECTRODE TIP FOR AN IMPLANTABLE CARDIAC ELECTRODE**
ELEKTRODENSPITZE FÜR EINE IMPLANTIERBARE HERZELEKTRODE
POINTE D'ÉLECTRODE POUR ÉLECTRODE CARDIAQUE IMPLANTABLE

(43) Date of publication of application: 08.03.2023
(73) Proprietor: BIOTRONIK SE & Co. KG, 12359 Berlin (DE)
(72) Inventor: Dohmen, Daniel, 238260 Singapore (SG); Zhen Fu Timothy, Bay, 760609 Singapore (SG)
(74) Representative: Biotronik Corporate Services SE

(56) References cited:
- US-A- 5 300 108
- US-A- 5 456 708
- US-A1- 2011 196 463
- US-A1- 2018 133 464

## Description

The present invention relates to an electrode tip for an implantable cardiac electrode according to the preamble of claim 1, to an implantable cardiac electrode comprising such an electrode tip according to the preamble of claim 9 and to methods for manufacturing such an electrode tip according to the preambles of claims 13 and 14.

In the field of medical technology, many different methods for sealing two elements against each other are known. To give an example, US 5,149,330 A describes a catheter capable of being converted from a single lumen catheter into a multilumen catheter. This catheter comprises a connector part that is insert-molded into the tubes of the catheter. The connector can be designed as standard Luer connector.

US 8,825,180 B2 describes a multi-conductor medical electrical lead comprising a connector located at a proximal end of the lead, one or more electrodes located at a distal end of the lead and a co-radial multi-conductor coil connecting the connector with the electrode(s). Here, a tip seal is insert-molded onto an electrode core prior to assembly into the conductor and is sealed with a silicone adhesive at the time of assembly.

US 9,901,725 B2 describes a high-pressure medical connector tubing assembly including a tube element having opposed tube ends and a passageway, an end element over-molded to at least one of the opposed tube ends, and a connector element having a connector hub defining a receiving cavity. Luer connector fittings may be insert-molded onto the ends of the tube.

US 10,207,091 B2 describes a force-directional nasal surgery dilatation device for use in a medical procedure such as sinuplasty, correction of deviated septum, and expansion of sinus cavities and nasal passages. An inflatable balloon is affixed to the distal end of a shaft for supporting and guiding the balloon into position for a nasal surgery procedure. Two tubes are contained inside of the shaft, wherein one defines a lumen for passage of a guide, and the other defines an inflation lumen. An inflation port, a guide port, and the shaft are connected in a hub. The inflation port is a tube generally bounded to a Luer connector, which receives the fluid to inflate the balloon. The Luer connector can be adhesive bonded or insert-molded onto the port tubing. US5456708A discloses a rotatable pin, screw-in type lead assembly for a cardiac pacemaker. US2018/133464A1 discloses an implantable lead with a helical screw.

When manufacturing an electrode tip for an implantable cardiac electrode, typically complicated manufacturing steps aiming at assembling a sealing into the small components of the electrode tip are necessary. For this purpose, an individually injection-molded sealing ring can be assembled with other parts of the electrode tip so as to employ its sealing functions. According to an alternative manufacturing method, a threaded joint is established between a shaft and a bushing of the electrode tip. A further possibility is to clamp an O-ring between two parts of the electrode tip in order to achieve sufficient sealing.

All of these conventional manufacturing methods require an additional assembly process for the individual parts. Since the individual parts are very small, the assembly process is not straightforward. To reduce the number of deficient products, assembly tolerances are needed for the individual components, making the individual components more expensive. Furthermore, if a threaded joint is established between two parts of the electrode tip, this threaded joint typically does not sufficiently seal the parts against each other. Furthermore, it has a comparatively high friction. Finally, the design of the electrode tip is limited, as one always needs some handling space for holding in position the additional parts to be assembled into the electrode tip.

Summarizing, the manufacturing processes of electrode tips known from prior art are time consuming and costly.

It is an object of the present invention to provide an electrode tip for an implantable cardiac electrode that exhibits sufficient sealing properties for sealing an implantable lead against a body fluid and that can be manufactured in a cheaper and easier way than electrode tips known from prior art.

This object is achieved with an electrode tip for an implantable cardiac electrode having the claim elements according to claim 1. Such an electrode tip comprises a housing, wherein the housing has an electrode lead feed section, an electrode lead receiving section and a sealing element arranged between the electrode lead feed section and the electrode lead receiving section. The sealing element serves for sealing the electrode lead feed section in a fluid tight manner against the electrode lead receiving section. This sealing is achieved when an electrode lead is arranged within the electrode lead feed section and the electrode lead receiving section and is surrounded by the sealing element. Such an arrangement of an electrode lead is given during the intended operation of the electrode tip. Then, the electrode lead is guided from the electrode lead feed section through the sealing member into the electrode lead receiving section.

Due to the established sealing, body fluids like blood are prevented from entering the electrode lead and from causing electrical shorts between individual conductors of the electrode lead. Furthermore, an undesired coagulation of blood is prevented. Thus, the sealing element serves for ensuring the functionality of the electrode lead and thus of the whole implantable cardiac electrode.

According to an aspect of the present invention, the sealing element consists of an injection-moldable material and constitutes a non-removable sealing portion. This sealing portion forms an integral part of the housing. Thus, the sealing element forms an integral part of the housing of the electrode tip. In contrast to prior art solutions, no separate manufacturing of a sealing element and co-assembling it with the housing of the electrode tip is necessary. Rather, the sealing element is directly produced at its intended site of operation, i.e., within the housing..

Thus, the presently claimed electrode tip can be manufactured by insert-molding a sealing into an already pre-manufactured component of the electrode tip. This allows a cost-efficient and automated manufacturing of the electrode tip. It opens up possibilities for new designs of leads and lead components. At the same time, the fluid tightness and the screwing mechanisms of active implantable cardiac electrodes can be improved by using the presently claimed electrode tip.

The presently claimed electrode tip does no longer require a complicated assembly process of a sealing ring into the other components of the electrode tip. Due to the possibility of injection molding the sealing element into the sealing chamber, more degrees of freedom result for the development of new electrode tip designs. In particular, the shape of the sealing member can be adjusted to the concrete needs of the respective electrode tip so as to improve the fluid tightness of the electrode tip. Furthermore, an improvement of the screwing mechanism of a screw of an active implantable electrode can be achieved based on a design that causes less friction than designs known from prior art. Finally, due to the presence of a sealing material also in the injection channel, interlocks are created during the molding process that ensure a reliable fixation of the sealing member to the remaining parts of the electrode tip and thus reduce the risk of a sealing member dislocation during the lifetime of the electrode tip.

In an embodiment, the sealing portion comprises a sealing chamber and an injection channel. The injection-moldable material is arranged within the sealing chamber and the injection channel. In this context, the injection channel extends from the sealing chamber to an exterior of the electrode tip. The injection channel serves for injecting the injection-moldable material in liquid form into the sealing chamber. After having set, the injection-moldable material forms the sealing element.

In an embodiment, the sealing element comprises sealing lips abutting against an electrode lead once guided from an exterior into an inside of the electrode tip. The sealing lips can have any desired shape so as to be easy to manufacture and to allow sufficient sealing against the outer circumference of the electrode lead to be guided through the sealing element.

In an embodiment, the injection-moldable material is chosen from the group consisting of liquid silicone rubber (LSR), thermoplastic elastomers (TPE), thermoplastic polyurethanes (TPU) and copolymers thereof. These materials are particularly appropriate to form the sealing member and to achieve a fluid tight sealing between the electrode lead feed section and the electrode lead receiving section of the housing.

In an embodiment, the housing comprises, in particularly essentially consists of, a housing material chosen from the group consisting of thermoplastic polymers, thermosetting plastics, ceramics, and bioinert metals such as stainless steel, titanium, and cobalt chromium.

In an embodiment, the housing material is biocompatible. Particular appropriate biocompatible materials are materials complying with the standard ISO 10993 and/or USP Class VI.

In an embodiment, the thermoplastic polymer is chosen from the group consisting of polyether ether ketones (PEEK), thermoplastic elastomers (TPE), thermoplastic polyurethanes (TPU), polyethylene terephthalate (PET), fluoropolymers, and copolymers of the precedingly mentioned polymers. Particularly appropriate fluoropolymers are polytetrafluoroethylene (PTFE), perfluoroalkoxy alkanes (PFA), ethylene tetrafluoroethylene copolymer (ETFE), and fluoroethylene propylene (FEP).

In an embodiment, the thermosetting plastics are chosen from the group consisting of polyester resins, polyurethanes (PUR), polyurea/polyurethane hybrids, parylene, polycarbonate (PC), polyethylene (PE), liquid crystal polymers, polypropylene (PP), polyphenylsulfone (PPSU), polyacetales (POM), vulcanized rubber, duroplast, epoxy resins, epoxy novolac resins, benzoxazines, polyimides, bismaleimides, cyanate esters, polycyanurates, furan resins, silicone, silicone resins, thiolyte, and vinyl ester resins.

In an embodiment, the housing material has a foam structure or a gel structure.

In an embodiment, the injection-moldable material used for manufacturing the sealing member is a different material than the housing material used for producing the housing of the electrode tip. Then, a particularly high sealing efficiency can be achieved by positioning the sealing member between the electrode lead feed section and the electrode lead receiving section.

In an embodiment, the sealing element comprises an undercut with respect to the sealing chamber. This undercut can be realized, e.g., by a portion of the sealing member being positioned within the injection channel. Since the injection channel does typically not extend over the whole outer circumference of the sealing chamber, the presence of the sealing material within the injection channel typically automatically leads to the presence of such an undercut. Such undercut prevents a rotational movement of the sealing member around an axis extending through a central opening of the sealing member (the central opening being intended to receive the electrode lead during operation of the electrode tip). The undercut typically also prevents an axial movement of the sealing member so that the sealing member is tightly kept in place. This enhances the sealing capabilities of the sealing member and results in a particularly efficient fluid tight sealing between the electrode lead feed section and the electrode lead receiving section due to the sealing member. Conventional sealing members such as O-rings do not comprise such an undercut and can dislocate during the lifetime of the electrode tip.

An undercut can also be realized by positioning the sealing member around a portion of the housing.

In an aspect, the present invention relates to an implantable cardiac electrode comprising an electrode tip according to the preceding explanations as well as an electrode lead arranged within the electrode tip. To be more precise, the electrode lead is arranged within the electrode lead feed section and the electrode lead receiving section and is surrounded by the sealing element. The sealing element has typically a ring-shaped appearance with a central opening through which the electrode lead can be guided so that the surface of the sealing element abuts an outer surface of the electrode lead.

In an embodiment, the implantable cardiac electrode is designed as an electrode that can be actively fixed. For this purpose, the electrode lead comprises a fixation screw received within the housing. The fixation screw it electrically conductive connected to the electrode lead and serves for fixing the electrode tip within the cardiac tissue. For this purpose, the fixation screw is typically turned like a screwdriver into the cardiac tissue of the patient at the desired implantation site. To achieve such fixation, a radial and axial movement of the fixation screw is necessary. The design of the electrode tip allows such radial and axial movement of the fixation screw.

In an embodiment, an electrically conductive connection between the fixation screw and the electrode lead is established within the electrode lead receiving section. For this purpose, a connecting sleeve or another connective element can be placed between the fixation screw and the electrode lead so as to realize the electrically conductive connection between these elements.

In an embodiment, the connecting sleeve is located around the electrode lead in a distal electrode lead section located within the electrode lead receiving space. The connecting sleeve typically extends along the whole circumference of the electrode lead.

In an embodiment, the housing comprises a protrusion or guiding pin that radially protrudes from an inner wall of the housing into the electrode lead receiving space. The protrusion controls an axial movement of the fixation screw along a longitudinal axis extending along a longitudinal extension direction of the housing. The protrusion does not restrict a radial movement of the fixation screw around the longitudinal axis. Thus, the protrusion serves for an axtraction of the screw so as to be able to get anchored in cardiac tissue. At the same time, it efficiently prevents the fixation screw from getting pulled out of the electrode lead receiving space through the distal end thereof. The protrusion is an integral part of the housing. Thus, it is not necessary to provide any additional element for restricting an axial movement of the fixation screw. Consequently, the number of components can be further reduced if the housing is designed according to this embodiment of the presently claimed invention.

In an aspect, the present invention relates to a method for manufacturing an electrode tip according to any of the preceding explanations. In this context, the method comprises the steps explained in the following.

First, a pre-manufactured housing is placed into a mold. This pre-manufactured housing can also be referred to as semi-finished housing. The housing has an electrode lead feed section, an electrode lead receiving section, and a sealing material receiving portion arranged between the electrode lead feed section and the electrode lead receiving section.

Afterwards, a liquid injection-moldable sealing material is injected into the sealing material receiving portion.

Finally, the sealing material is allowed to set. Thereby, the sealing element is formed within the sealing material receiving portion. This sealing element forms an integral part of the housing and serves for a fluid tight sealing between the electrode lead feed section and the electrode receiving section once an electrode lead is guided from the electrode lead feed section through an opening within the sealing element towards the electrode lead receiving section.

In an embodiment, the sealing material receiving portion comprises a sealing chamber and an injection channel. The injection channel extends from the sealing chamber to an exterior of the housing so that the sealing chamber is accessible from the exterior of the housing. In this embodiment, the injection-moldable material is injected through the injection channel into the sealing chamber.

In an embodiment, the sealing portion or the sealing chamber comprises a core pin for defining sealing lips of the sealing element to be formed. The core pin can have any desired shape to allow the manufacturing of the sealing element having sealing lips achieving sufficient sealing against an electrode lead to be guided through the sealing element.

In an aspect, the present invention relates to a further method for manufacturing an electrode tip according to the preceding explanations. This method comprises the steps explained in the following.

A liquid injection-moldable housing material and a liquid injection-moldable sealing material are injected in a two-component injection molding process into a mold. This two-component injection molding process can also be denoted as 2K injection molding. As a result, the housing material forms a housing having an electrode lead feed section, an electrode lead receiving section, and a sealing material receiving portion arranged between the electrode lead feed section and the electrode lead receiving section. The sealing material is present in the sealing material receiving portion.

The sealing material is allowed to set. Thereby, a sealing member is formed within the sealing material receiving portion. Likewise, the housing material is allowed to set so as to be able to form the precedingly explained components of the housing.

In an embodiment, the sealing material receiving portion comprises a sealing chamber and an injection channel. The injection channel extends from the sealing chamber to an exterior of the housing so that the sealing chamber is accessible from the exterior of the housing. In this embodiment, the injection-moldable material is present in the injection channel and in the sealing chamber.

In an embodiment, the sealing portion or the sealing chamber comprises a core pin for defining sealing lips of the sealing element to be formed. Reference is made to the explanations given above with respect to the shape of the core pin and the function of the sealing lips.

Both manufacturing methods are particularly simple since they do not require the assembly and manipulation of particularly small components of the electrode tip. Rather, the required components can be produced in separate injection molding steps or in a combined two-component injection molding step. Furthermore, the housing of the electrode tip can be manufactured by any desired mechanical method if the first of the explained method is applied. This significantly reduces the complexity and costs of the manufacturing process.

All embodiments explained with respect to the electrode tip can be combined in any desired way and can be transferred either individually or in any arbitrary combination to the described implantable cardiac electrode and to the described methods. Likewise, all embodiments of the implantable cardiac electrode can be combined in any desired way and can be transferred either individually or in any arbitrary combination to the electrode tip and the described methods. Finally, all embodiments of the described methods can be combined in any desired way and can be transferred either individually or in any arbitrary combination to the electrode tip, to the implantable cardiac electrode and to the respective other method.

Further details of aspects of the present invention will be explained in the following with respect to exemplary embodiments and accompanying Figures. In the Figures:
- Fig. 1A: is a first schematic longitudinal section through an electrode tip of an embodiment of an implantable cardiac electrode;
- Fig. 1B: is a second schematic longitudinal section through the electrode tip of Fig. 1A, wherein the longitudinal section is rotated by 90° with respect to the depiction of Fig. 1A;
- Fig. 2A: is a schematic longitudinal section through a first embodiment of an electrode tip;
- Fig. 2B: is a schematic perspective view onto the electrode tip of Fig. 2A;
- Fig. 2C: is a schematic longitudinal section through a second embodiment of an electrode tip;
- Fig. 2D: is a schematic perspective view onto the electrode tip of Fig. 2C;
- Fig. 3A: is a schematic cross section through a first embodiment of a sealing member; and
- Fig. 3B: is a schematic cross section through a second embodiment of a sealing member.

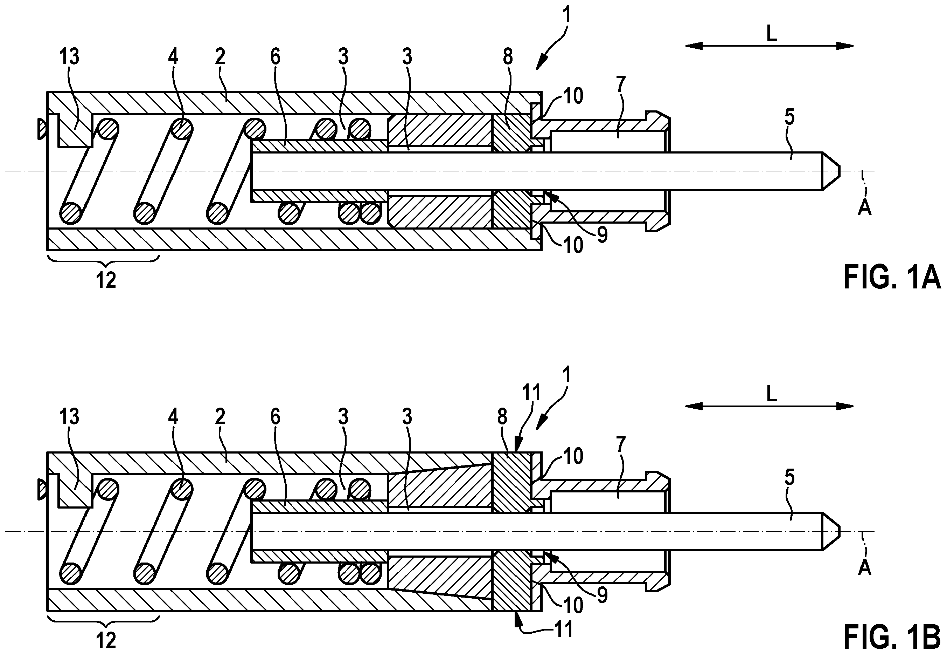

Fig. 1A shows a longitudinal section through an electrode tip 1 of an implantable cardiac electrode. The electrode tip 1 comprises a housing 2 extending along a longitudinal extension direction L and comprises a longitudinal axis A.

The housing 2 defines an electrode lead receiving space 3 in its interior. The electrode lead receiving space 3 serves for housing a fixation screw 4. The fixation screw 4 is intended to be turned into and thus anchored within cardiac tissue of a patient to whom the implantable cardiac electrode is to be implanted. The fixation screw 4 serves as first electrode pole. For this purpose, it is connected to an electrode lead 5 via an electrically conductive connecting sleeve 6.

The electrode lead 5 is guided from an exterior of the housing 2 through an electrode lead feed space 7 and through a sealing element 8 into the electrode lead receiving space 3. For this purpose, the electrode lead 5 is guided through a central bore 9 of the sealing element 8. The sealing element 8 is arranged within a sealing chamber 10 serving as sealing material receiving space.

It is possible to place a sleeve-like shaped second electrode pole in a distal end region 12 of the housing 2. In such a case, this second electrode pole is connected to a distinct electric lead of the electrode lead 5, which is, however, not shown in Fig. 1A.

The sealing member 8 serves for closing a proximal end of the electrode lead receiving space 3 against the electrode lead feed space 7 in a fluid tight manner. The distal end of the electrode lead receiving space 3 is open so as to allow the fixation screw 4 to exit the electrode lead receiving space 3 and to contact the cardiac tissue in which it is to be anchored.

The housing 2 comprises a protrusion 13 extending from an inner wall of the housing 2 into the electrode lead receiving space 3 towards the longitudinal axis A in a radial manner. This protrusion 13 controls an axial movement of the fixation screw 4 along the longitudinal axis A and a rotation of the fixation screw 4 about the longitudinal axis A.

Fig. 1B shows another longitudinal section through the electrode tip 1 already shown in Fig. 1A. In Fig. 1B, the longitudinal section is rotated by 90° with respect to the longitudinal section of the depiction of Fig. 1A. In this and in all following Figures, the same numeral references will be used for similar elements.

Since many elements of the electrode tip 1 are arranged in a rotationally symmetric way, there are only small deviations between the depiction of Fig. 1A and the depiction of Fig. 1B. The most prominent difference is the visibility of two injection channels 11 through which an injection moldable material can be inserted from an exterior of the housing 2 into the sealing chamber 10.

Upon manufacturing the electrode tip 1, an injection-moldable sealing material is inserted by injection molding through the injection channels 11 into the sealing chamber 10. Afterwards, the sealing material is allowed to set. Then, it forms the sealing element 8 located both within the sealing chamber 10 and the injection channels 11. Since the injection channels 11 do not extend over the whole outer circumference of the sealing chamber 10, an undercut of the sealing element 8 with respect to the sealing chamber 10 is realized. This undercut restricts the movement of the sealing element 8 within the sealing chamber 10 and thus prevents an undesired dislocation of the sealing element 8 during operation of the electrode tip 1.

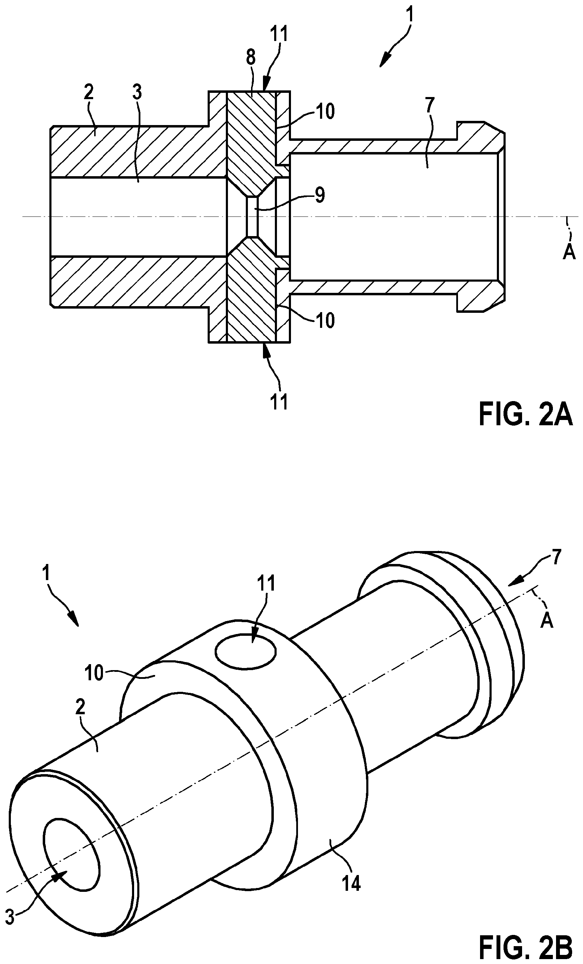

Fig. 2A is a schematic longitudinal section through an embodiment of an electrode tip 1. The electrode tip 1 comprises a housing 2 defining an electrode lead receiving space 3 and an electrode lead feed space 7. The electrode lead receiving space 3 and the electrode lead feed space 7 are arranged such that an electrode lead can be guided from the electrode lead feed space 7 through a central bore 9 of a sealing element 8 into the electrode lead receiving space 3. The sealing member 8 is injection molded into a sealing chamber 10 through two injection channels 11.

The nature of these injection channels 11 can be seen in more detail in Fig. 2B, representing a perspective view onto the electrode tip 1 of Fig. 2A. Here, the upper injection channel 11 is visible through which the sealing material is injection-molded into the sealing chamber 10 (cf. Fig. 2A) so as to form the sealing member 8 within the sealing chamber and the injection channels 11. Since both the upper injection channel 11 and the lower injection channel 11 (not visible in Fig. 2B, but in Fig. 2A) are identical in construction, it is apparent from Fig. 2B that these injection channels 11 only make up a small portion of the outer circumference 14 of the sealing chamber 10. Consequently, it will not be possible for the sealing member 8 to move radially around the longitudinal axis A after having set. The injection channels 11 act as undercut for the sealing member 8 with respect to the sealing chamber 10.

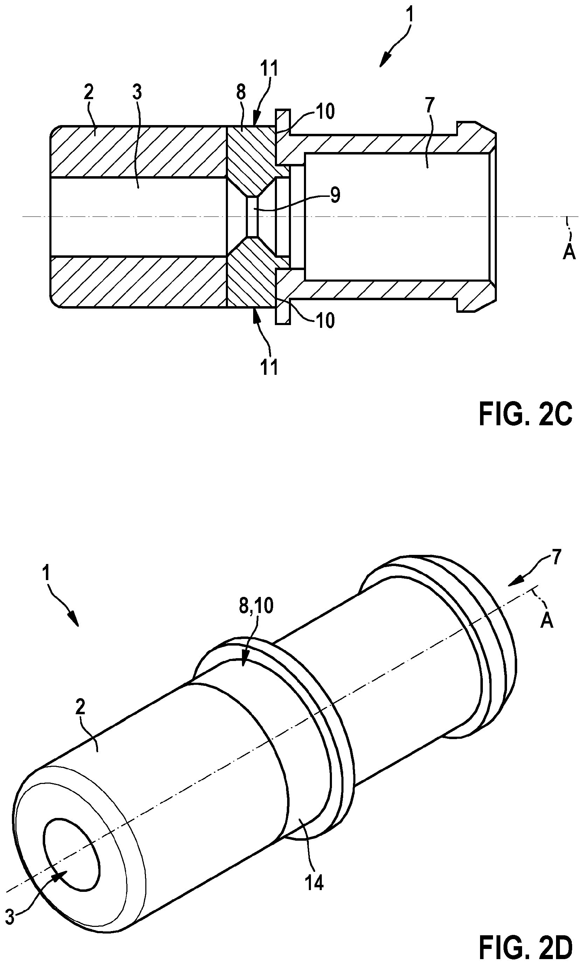

Fig. 2C is a schematic longitudinal section through another embodiment of an electrode tip 1. Since this embodiment is quite similar to the embodiment shown in Figures 2A and 2B, reference is made to the explanations given above. In the following, only the differences will be explained in detail.

In contrast to the embodiment shown in Fig. 2A, the electrode tip 1 of Fig. 2C does not comprise a sealing chamber, but rather a sealing material receiving portion 10. Here, the sealing element 8 is arranged without being encompassed by a wall of the housing 2. Rather, as can be seen from Fig. 2D, the sealing material of the sealing element 8 is present on the whole outer circumference 14 of the sealing portion 10. Therefore, this embodiment does not comprise injection channels in the narrower sense. Rather, the individual sections of the outer circumference 14 of the sealing portion 10 cannot be distinguished from each other as in case of the embodiment shown in Figures 2A and 2B.

However, the sealing properties of sealing element 8 of the embodiment shown in Figures 2C and 2D are identical to the sealing properties of the sealing element 8 of the electrode tip 1 shown in Figures 2A and 2B.

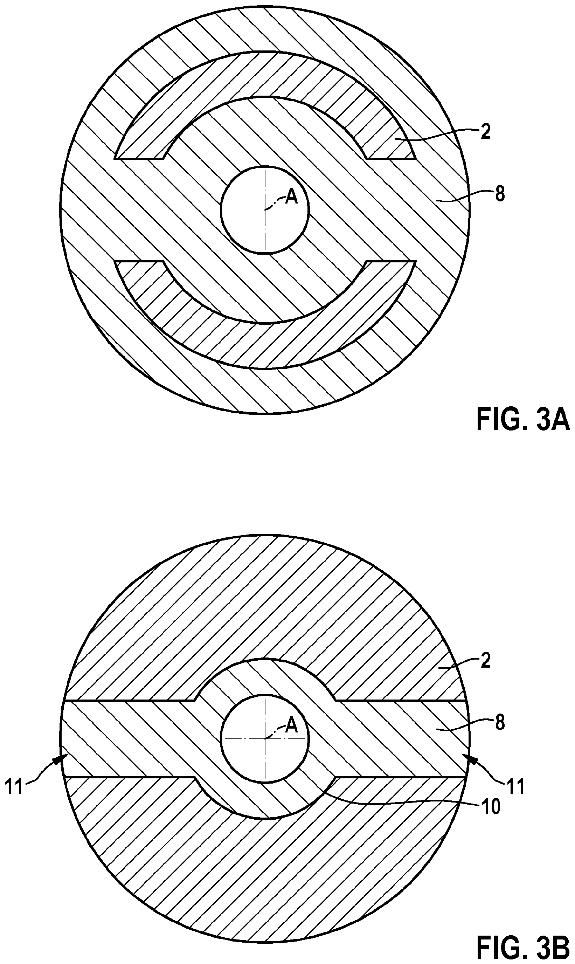

Fig. 3A shows a cross-section through a first embodiment of a sealing element 8 comprising an undercut. Here, the injection moldable material forming the sealing element 8 is injection molded around a part of the housing 2. Then, the housing 2 serves as reinforcement for the sealing element 8 and efficiently restricts undesired movements of the sealing element 8 about the axis A.

Fig. 3B shows a cross-section through a second embodiment of a sealing element 8 comprising an undercut. This sealing element 8 corresponds to the sealing element used in the embodiments shown in Figures 1A, 1B, 2A and 2B. Here, the sealing material is present in a sealing chamber 10 and two injection channels 11. Of course, any desired number of injection channels 11 may be used. The two injection channels 11 depicted in Fig. 3B are only understood to be exemplarily.

## Claims

1. Electrode tip for an implantable cardiac electrode, wherein the electrode tip (1) comprises a housing (2) having an electrode lead feed section (7), an electrode lead receiving section (3) and a sealing element (8) arranged between the electrode lead feed section (7) and the electrode lead receiving section (3), wherein the sealing element (8) serves for sealing the electrode lead feed section (7) in a fluid tight manner against the electrode lead receiving section (3), when an electrode lead (5) is arranged within the electrode lead feed section (7) and the electrode lead receiving section (3) and is surrounded by the sealing element (8), wherein the sealing element (8) consists of an injection-moldable material;
**characterized**
**in that** the sealing element (8) constitutes a non-removable sealing portion (10, 11) forming an integral part of the housing (2).

2. Electrode tip according to claim 1, wherein the sealing portion (10, 11) comprises a sealing chamber (10) and an injection channel (11), wherein the sealing chamber (10) and the injection channel (11) are filled with the injection-moldable material, wherein the injection channel (11) extends from the sealing chamber (10) to an exterior of the electrode tip (1) and serves for injecting the injection-moldable material in liquid form into the sealing chamber (10), the injection-moldable material forming the sealing element (8) after setting.

3. Electrode tip according to claim 1 or 2, wherein the inj ection-moldable material is chosen from the group consisting of liquid silicone rubber, thermoplastic elastomers, thermoplastic polyurethanes, and copolymers thereof.

4. Electrode tip according to any of the preceding claims, wherein the housing (2) comprises a housing material chosen from the group consisting of thermoplastic polymers, thermosetting plastics, ceramics, and bioinert metals.

5. Electrode tip according to claim 4, wherein the thermoplastic polymers are chosen from the group consisting of polyether ether ketones, thermoplastic elastomers, thermoplastic polyurethanes, polyethylene terephthalate, fluoropolymers, and copolymers thereof.

6. Electrode tip according to claim 4 or 5, wherein the thermosetting plastics are chosen from the group consisting of polyester resins, polyurethanes, polyurea/polyurethane hybrids, parylene, polycarbonate, polyethylene, liquid crystal polymers, polypropylene, polyphenylsulfone, polyacetales, vulcanized rubber, duroplast, epoxy resins, epoxy novolac resins, benzoxazines, polyimides, bismaleimides, cyanate esters, polycyanurates, furan resins, silicone, silicone resins, thiolyte, and vinyl ester resins.

7. Electrode tip according to any of the claims 4 to 6, wherein the injection-moldable material is a different material than the housing material.

8. Electrode tip according to any of the preceding claims, wherein the sealing element (8) comprises an undercut with respect to the sealing chamber (10).

9. Implantable cardiac electrode comprising an electrode tip (1) according to any of the preceding claims and an electrode lead (5) arranged within the electrode lead feed section (7) and the electrode lead receiving section (3) and being surrounded by the sealing element (8).

10. Implantable cardiac electrode according to claim 9, wherein the cardiac electrode comprises a fixation screw (4) received within the housing (2), wherein the fixation screw (4) is electrically conductive connected to the electrode lead (5) and serves for fixing the electrode tip (1) within cardiac tissue.

11. Implantable cardiac electrode according to claim 10, wherein an electrically conductive connection between the fixation screw (4) and the electrode lead (5) is established within the electrode lead receiving section (3).

12. Implantable cardiac electrode according to claim 10 or 11, wherein the housing (2) comprises a protrusion (13) radially protruding from a wall of the housing (2) into the electrode lead receiving space (3), wherein the protrusion (13) limits an axial movement of the fixation screw (4) along a longitudinal axis (A) extending along a longitudinal extension direction (L) of the housing (2), wherein the protrusion (13) does not limit a radial movement of the fixation screw (4) around the longitudinal axis (A).

13. Method for manufacturing an electrode tip (1) according to any of claims 1 to 8, comprising the following steps:
a) placing a pre-manufactured housing (2) into a mold, the housing (2) having an electrode lead feed section (7), an electrode lead receiving section (3), and a sealing material receiving portion (10, 11) arranged between the electrode lead feed section (7) and the electrode lead receiving section (3);
b) injecting a liquid inj ection-moldable sealing material into the sealing material receiving portion (10, 11); and
c) allowing the sealing material to set, thereby forming a non-removable sealing element (8) within the sealing material receiving portion (10, 11), the sealing element (8) forming an integral part of the housing (2).

14. Method for manufacturing an electrode tip (1) according to any of claims 1 to 8, comprising the following steps:
a) injecting a liquid injection-moldable housing material and a liquid injection-moldable sealing material in a two-component injection molding process into a mold so as to form a housing (2) having an electrode lead feed section (7), an electrode lead receiving section (3), and a sealing material receiving portion (10, 11) arranged between the electrode lead feed section (7) and the electrode lead receiving section (3); and
b) allowing the sealing material to set, thereby forming a sealing element (8) within the sealing material receiving portion (10, 11).

15. Method according to claim 13 or 14, wherein the sealing material receiving portion (10, 11) comprises a sealing chamber (10) and an injection channel (11), wherein the injection channel (11) extends from the sealing chamber (10) to an exterior of the housing (2), wherein the sealing material is present in the injection channel (11) and in the sealing chamber (10).

## Patentansprüche

1. Elektrodenspitze für eine implantierbare Herzelektrode, wobei die Elektrodenspitze (1) ein Gehäuse (2) mit einem Elektrodenleitungszuführungsabschnitt (7), einem Elektrodenleitungsaufnahmeabschnitt (3) und einem zwischen dem Elektrodenleitungszuführungsabschnitt (7) und dem Elektrodenleitungsaufnahmeabschnitt (3) angeordneten Dichtungselement (8) aufweist, wobei das Dichtungselement (8) dazu dient, den Elektrodenleitungszuführungsabschnitt (7) fluiddicht gegen den Elektrodenleitungsaufnahmeabschnitt (3) abzudichten, wenn eine Elektrodenleitung (5) innerhalb des Elektrodenleitungszuführungsabschnitts (7) und des Elektrodenleitungsaufnahmeabschnitts (3) angeordnet und von dem Dichtungselement (8) umgeben ist, wobei das Dichtungselement (8) aus einem spritzgießbaren Material besteht;
**dadurch gekennzeichnet**
**dass** das Dichtungselement (8) einen nicht entfernbaren Dichtungsabschnitt (10, 11) darstellt, der einen integralen Bestandteil des Gehäuses (2) bildet.

2. Elektrodenspitze nach Anspruch 1, wobei der Dichtungsabschnitt (10, 11) eine Dichtungskammer (10) und einen Injektionskanal (11) aufweist, wobei die Dichtungskammer (10) und der Injektionskanal (11) mit dem spritzgießbaren Material gefüllt sind, wobei sich der Injektionskanal (11) von der Dichtungskammer (10) zu einer Außenseite der Elektrodenspitze (1) erstreckt und dazu dient, das spritzgießbare Material in flüssiger Form in die Dichtungskammer (10) zu injizieren, wobei das spritzgießbare Material nach dem Aushärten das Dichtungselement (8) bildet.

3. Elektrodenspitze nach Anspruch 1 oder 2, wobei das spritzgießbare Material aus der Gruppe ausgewählt ist, die aus flüssigem Silikonkautschuk, thermoplastischen Elastomeren, thermoplastischen Polyurethanen und deren Copolymeren besteht.

4. Elektrodenspitze nach einem der vorhergehenden Ansprüche, wobei das Gehäuse (2) ein Gehäusematerial umfasst, das aus der Gruppe ausgewählt ist, die aus thermoplastischen Polymeren, duroplastischen Kunststoffen, Keramiken und bioinerten Metallen besteht.

5. Elektrodenspitze nach Anspruch 4, wobei die thermoplastischen Polymere aus der Gruppe ausgewählt sind, die aus Polyetheretherketonen, thermoplastischen Elastomeren, thermoplastischen Polyurethanen, Polyethylenterephthalat, Fluorpolymeren und deren Copolymeren besteht.

6. Elektrodenspitze nach Anspruch 4 oder 5, wobei die duroplastischen Kunststoffe ausgewählt sind aus der Gruppe bestehend aus Polyesterharzen, Polyurethanen, Polyharnstoff/Polyurethan-Hybriden, Parylen, Polycarbonat, Polyethylen, Flüssigkristallpolymeren, Polypropylen, Polyphenylsulfon, Polyacetalen, vulkanisierter Kautschuk, Duroplast, Epoxidharzen, Epoxid-Novolakharzen, Benzoxazinen, Polyimiden, Bismaleimiden, Cyanatestern, Polycyanuraten, Furanharzen, Silicon, Siliconharzen, Thiolyt und Vinylesterharzen.

7. Elektrodenspitze nach einem der Ansprüche 4 bis 6, wobei das spritzgießbare Material ein anderes Material ist als das Gehäusematerial.

8. Elektrodenspitze nach einem der vorhergehenden Ansprüche, wobei das Dichtungselement (8) eine Hinterschneidung gegenüber der Dichtungskammer (10) aufweist.

9. Implantierbare Herzelektrode mit einer Elektrodenspitze (1) nach einem der vorhergehenden Ansprüche und einer Elektrodenleitung (5), die innerhalb des Elektrodenleitungszuführungsabschnitts (7) und des Elektrodenleitungsaufnahmeabschnitts (3) angeordnet und von dem Dichtungselement (8) umgeben ist.

10. Implantierbare Herzelektrode nach Anspruch 9, wobei die Herzelektrode eine im Gehäuse (2) aufgenommene Fixierschraube (4) aufweist, wobei die Fixierschraube (4) elektrisch leitend mit der Elektrodenleitung (5) verbunden ist und zur Fixierung der Elektrodenspitze (1) im Herzgewebe dient.

11. Implantierbare Herzelektrode nach Anspruch 10, wobei eine elektrisch leitende Verbindung zwischen der Fixierschraube (4) und der Elektrodenleitung (5) innerhalb des Elektrodenleitungsaufnahmeabschnitts (3) eingerichtet ist.

12. Implantierbare Herzelektrode nach Anspruch 10 oder 11, wobei das Gehäuse (2) einen Vorsprung (13) aufweist, der von einer Wand des Gehäuses (2) radial in den Elektrodenleitungsaufnahmeabschnitt (3) hineinragt, wobei der Vorsprung (13) eine axiale Bewegung der Fixierschraube (4) entlang einer Längsachse (A) begrenzt, die sich entlang einer Längserstreckungsrichtung (L) des Gehäuses (2) erstreckt, wobei der Vorsprung (13) eine radiale Bewegung der Fixierschraube (4) um die Längsachse (A) nicht begrenzt.

13. Verfahren zur Herstellung einer Elektrodenspitze (1) nach einem der Ansprüche 1 bis 8, umfassend die folgenden Schritte:
a) Einsetzen eines vorgefertigten Gehäuses (2) in eine Form, wobei das Gehäuse (2) einen Elektrodenleitungszuführungsabschnitt (7), einen Elektrodenleitungsaufnahmeabschnitt (3) und einen zwischen dem Elektrodenleitungszuführungsabschnitt (7) und dem Elektrodenleitungsaufnahmeabschnitt (3) angeordneten Dichtungsmaterialaufnahmeabschnitt (10, 11) aufweist;
b) Einspritzen eines flüssigen, spritzgießbaren Dichtungsmaterials in den Dichtungsmaterialaufnahmeabschnitt (10, 11); und
c) Aushärtenlassen des Dichtungsmaterials, wodurch ein nicht entfernbares Dichtungselement (8) innerhalb des Dichtungsmaterialaufnahmeabschnitts (10, 11) gebildet wird, wobei das Dichtungselement (8) einen integralen Bestandteil des Gehäuses (2) bildet.

14. Verfahren zur Herstellung einer Elektrodenspitze (1) nach einem der Ansprüche 1 bis 8, umfassend die folgenden Schritte:
a) Einspritzen eines flüssigen spritzgießbaren Gehäusematerials und eines flüssigen spritzgießbaren Dichtungsmaterials in einem Zweikomponenten-Spritzgießverfahren in eine Form, um ein Gehäuse (2) zu bilden, das einen Elektrodenleitungszuführungsabschnitt (7), einen Elektrodenleitungsaufnahmeabschnitt (3) und einen zwischen dem Elektrodenleitungszuführungsabschnitt (7) und dem Elektrodenleitungsaufnahmeabschnitt (3) angeordneten Dichtungsmaterialaufnahmeabschnitt (10, 11) aufweist; und
b) Aushärtenlassen des Dichtungsmaterials, wodurch ein Dichtungselement (8) innerhalb des Dichtungsmaterialaufnahmeabschnitts (10, 11) gebildet wird.

15. Verfahren nach Anspruch 13 oder 14, wobei der Dichtungsmaterialaufnahmeabschnitt (10, 11) eine Dichtungskammer (10) und einen Injektionskanal (11) umfasst, wobei sich der Injektionskanal (11) von der Dichtungskammer (10) zu einer Außenseite des Gehäuses (2) erstreckt, wobei das Dichtungsmaterial in dem Injektionskanal (11) und in der Dichtungskammer (10) vorhanden ist.

## Revendications

1. Pointe d'électrode pour une électrode cardiaque implantable, dans laquelle la pointe d'électrode (1) comprend un boîtier (2) avec une section d'alimentation du fil d'électrode (7), une section de réception du fil d'électrode (3) et un élément d'étanchéité (8) disposé entre la section d'alimentation du fil d'électrode (7) et la section de réception du fil d'électrode (3), dans lequel l'élément d'étanchéité (8) sert à sceller la section d'alimentation du fil d'électrode (7) de manière étanche aux fluides contre la section de réception du fil d'électrode (3), lorsqu'un fil d'électrode (5) est disposé à l'intérieur de la section d'alimentation du fil d'électrode (7) et de la section de réception du fil d'électrode (3) et est entouré par l'élément d'étanchéité (8), dans lequel l'élément d'étanchéité (8) est constitué d'un matériau moulable par injection ;
**caractérisé**
**en ce que** l'élément d'étanchéité (8) constitue une partie d'étanchéité non amovible (10, 11) faisant partie intégrante du boîtier (2).

2. Pointe d'électrode selon la revendication 1, dans laquelle la partie d'étanchéité (10, 11) comprend une chambre d'étanchéité (10) et un canal d'injection (11), dans lesquels la chambre d'étanchéité (10) et le canal d'injection (11) sont remplis de matériau moulable par injection, dans lequel le canal d'injection (11) s'étend de la chambre d'étanchéité (10) vers l'extérieur de la pointe d'électrode (1) et sert à injecter le matériau moulable par injection sous forme liquide dans la chambre d'étanchéité (10), le matériau injectable formant l'élément d'étanchéité (8) après la prise.

3. Pointe d'électrode selon la revendication 1 ou 2, dans laquelle le matériau moulable par injectionest choisi dans le groupe constitué du caoutchouc de silicone liquide, des élastomères thermoplastiques, des polyuréthanes thermoplastiques et des copolymères de celui-ci.

4. Pointe d'électrode selon l'une quelconque des revendications précédentes, dans laquelle le boîtier (2) comprend un matériau choisi dans le groupe constitué de polymères thermoplastiques, de plastiques thermodurcissables, de céramiques et de métaux bioinertes.

5. Pointe d'électrode selon la revendication 4, dans laquelle les polymères thermoplastiques sont choisis dans le groupe constitué des polyéther-éther-cétones, des élastomères thermoplastiques, des polyuréthanes thermoplastiques, du polyéthylène téréphtalate, des fluoropolymères et des copolymères de celui-ci.

6. Pointe d'électrode selon la revendication 4 ou 5, dans laquelle les plastiques thermodurcissables sont choisis dans le groupe constitué de résines de polyester, des polyuréthanes, des hybrides polyurée/polyuréthane, du parylène, du polycarbonate, du polyéthylène, des polymères à cristaux liquides, du polypropylène, du polyphénylsulfone, des polyacétales, du caoutchouc vulcanisé, du duroplast, des résines époxy, des résines époxy novolac, des benzoxazines, des polyimides, des bismaléimides, des esters de cyanate, des polycyanurates, des résines furaniques, du silicone, des résines de silicone, du thiolyte et des résines d'ester vinylique.

7. Pointe d'électrode selon l'une des revendications 4 à 6, dans laquelle le matériau moulable par injectionest un matériau différent de celui du boîtier.

8. Pointe d'électrode selon l'une des revendications précédentes, dans laquelle l'élément d'étanchéité (8) comporte une contre-dépouille par rapport à la chambre d'étanchéité (10).

9. Electrode cardiaque implantable comprenant une pointe d'électrode (1) selon l'une quelconque des revendications précédentes et un fil d'électrode (5) disposé à l'intérieur de la section d'alimentation du fil d'électrode (7) et de la section de réception du fil d'électrode (3) et entouré par l'élément d'étanchéité (8).

10. Electrode cardiaque implantable selon la revendication 9, dans laquelle l'électrode cardiaque comprend une vis de fixation (4) reçue à l'intérieur du boîtier (2), dans laquelle la vis de fixation (4) est connectée par conduction électrique au fil de l'électrode (5) et sert à fixer la pointe de l'électrode (1) à l'intérieur du tissu cardiaque.

11. Electrode cardiaque implantable selon la revendication 10, dans laquelle une connexion électriquement conductrice entre la vis de fixation (4) et la sonde d'électrode (5) est établie à l'intérieur de la section de réception de la sonde d'électrode (3).

12. Electrode cardiaque implantable selon la revendication 10 ou 11, dans laquelle le boîtier (2) comprend une saillie (13) faisant radialement saillie à partir d'une paroi du boîtier (2) dans l'espace de réception de la sonde d'électrode (3), dans laquelle la saillie (13) limite un mouvement axial de la vis de fixation (4) le long d'un axe longitudinal (A) s'étendant le long d'une direction d'extension longitudinale (L) du boîtier (2), dans laquelle la saillie (13) ne limite pas un mouvement radial de la vis de fixation (4) autour de l'axe longitudinal (A).

13. Procédé de fabrication d'une pointe d'électrode (1) selon l'une des revendications 1 à 8, comprenant les étapes suivantes :
a) placer un boîtier préfabriqué (2) dans un moule, le boîtier (2) ayant une section d'alimentation du fil d'électrode (7), une section de réception de fil d'électrode (3), et une partie de réception de matériau d'étanchéité (10, 11) disposée entre la section d'alimentation du fil d'électrode (7) et la section de réception du fil d'électrode (3) ;
b) injecter un matériau d'étanchéité liquide moulable par injection dans la partie de réception du matériau d' étanchéité (10, 11) ; et
c) laisser le matériau d'étanchéité se fixer, formant ainsi un élément d'étanchéité non amovible (8) à l'intérieur de la partie de réception du matériau d'étanchéité (10, 11), l'élément d'étanchéité (8) faisant partie intégrante du boîtier (2).

14. Procédé de fabrication d'une pointe d'électrode (1) selon l'une des revendications 1 à 8, comprenant les étapes suivantes :
a) injecter un matériau de boîtier liquide moulable par injection et un matériau d'étanchéité liquide moulable par injection dans un processus de moulage par injection à deux composants dans un moule de manière à former un boîtier (2) ayant une section d'alimentation du fil d'électrode (7), une section de réception du fil d'électrode (3) et une partie de réception de matériau d'étanchéité (10, 11) disposée entre la section d'alimentation du fil d'électrode (7) et la section du réception de fil d'électrode (3) ; et
b) laisser le matériau d'étanchéité se fixer, formant ainsi un élément d'étanchéité (8) à l'intérieur la partie de réception du matériau d'étanchéité (10, 11).

15. Procédé selon la revendication 13 ou 14, dans lequel la partie de réception du matériau d'étanchéité (10, 11) comprend une chambre d'étanchéité (10) et un canal d'injection (11), dans lequel le canal d'injection (11) s'étend de la chambre d'étanchéité (10) vers l'extérieur du boîtier (2), dans lequel le matériau d'étanchéité est présent dans le canal d'injection (11) et dans la chambre d'étanchéité (10).
